# EUROPÄISCHE PATENTSCHRIFT

(11) **EP 3 250 275 B1**
(45) Veröffentlichungstag und Bekanntmachung des Hinweises auf die Patenterteilung: **07.08.2019**
(21) Anmeldenummer: 16709964.7
(22) Anmeldetag: 27.01.2016
(51) Int. Cl.: A61M 13/00, A61M 16/10, G05D 23/24

(54) **VORRICHTUNG ZUR REGELUNG DER TEMPERATUR DES GASSTROMS BEI MEDIZINTECHNISCHEN VORRICHTUNGEN**
DEVICE FOR CONTROLLING THE TEMPERATURE OF THE GAS FLOW IN MEDICAL DEVICES
DISPOSITIF POUR RÉGULER LA TEMPÉRATURE DU FLUX GAZEUX POUR DES DISPOSITIFS MÉDICO-TECHNIQUES

(30) Priorität: 27.01.2015 DE 102015000845
(43) Veröffentlichungstag der Anmeldung: 06.12.2017
(73) Patentinhaber: W.O.M. World of Medicine GmbH, 10587 Berlin (DE)
(72) Erfinder: MENZEL, Felix, 10551 Berlin (DE); ZEYSSIG, Andreas, 10245 Berlin (DE); KÖRNER, Johannes, 88250 Weingarten (DE)
(74) Vertreter: Jungblut & Seuss
(86) Internationale Anmeldenummer: PCT/DE2016/000027
(87) Internationale Veröffentlichungsnummer: WO 2016/119773

(56) Entgegenhaltungen:
- WO-A2-2013/098397
- DE-A1- 19 545 719
- US-A- 3 789 190
- US-A- 5 411 474
- US-A1- 2004 221 844
- US-A1- 2010 312 416
- Felix Menzel: "Beobachtergestützte Regelung einer Gasheizung in der Minimal-Invasiven-Medizin (kurz MIM)", , 12. Mai 2015 (2015-05-12), Seiten 1-21, XP055270174, Gefunden im Internet: URL:http://spectronet.de/story_docs/vortra ege_2015/150512_matlab_expo_2015/150512_18 _menzel_wom.pdf [gefunden am 2016-05-03]

## Beschreibung

Die vorliegende Erfindung betrifft eine medizintechnische Vorrichtung mit verbesserter Regelung der Gastemperatur, zur Verwendung beispielsweise. im Rahmen der Laparoskopie oder der Beatmung.

Im Rahmen verschiedener medizinischer Prozeduren werden Gase in das Körperinnere eingeführt. Beispielhaft genannte sei die Laparoskopie, bei der bisher während eines therapeutischen Eingriffs Gase (z. B. CO₂) in das Abdomen geführt werden. Im Rahmen dieser Prozeduren wird das zugeführte Gas in der Regel beheizt, so dass das in das Körperinnere eintretende Gas annähernd Körpertemperatur hat, da sowohl zu kalte, als auch zu warme Gase zu Schmerzreizen des Patienten führen. Es ist daher die Messung und die Regelung der Gastemperatur von besonderer Bedeutung. Typischerweise werden die für derartige Prozeduren benutzten Gasleitungen mit Temperatursensoren versehen, die eine entsprechende Temperaturregelung ermöglichen sollen. Der Einsatz derartiger separater Sensoren ist unter anderem deswegen nachteilig, weil diese zusätzliche Kosten verursachen. Da die dazugehörigen Schläuche Einwegartikel sind, sollen diese Kosten möglichst vermieden werden. Eine andere Möglichkeit der Temperaturmessung stellt die Messung der Temperatur des Heizdrahtes dar. Zwischen der Gastemperatur am Ausgang des Schlauches und der Temperatur des Heizdrahtes besteht zwar ein Zusammenhang, dieser ist jedoch von einer Anzahl von Einflussgrößen abhängig, wie z.B. dem Volumenstrom des Gases, der Gasart, der Heizleistung, der Geometrie und dem Material des Schlauches sowie der Außentemperatur, um nur einige der Faktoren zu nennen.

US 2004/0221844 beschreibt eine klassische Beatmungsvorrichtung mit Temperaturregelung des Beatmungsgases. WO2013/098397 A2 beschreibt eine Vorrichtung zum Erzeugen eines Aerosols für die Inhalation mit Temperaturregelung.

Vor diesem Hintergrund stellt sich die Aufgabe, eine Vorrichtung mit verbesserter Temperaturregelung anzugeben, welche die vorgenannten Nachteile überwindet. Zur Lösung des Problems wird die Vorrichtung gemäß Anspruch 1 vorgeschlagen. Vorteilhafte Ausgestaltungen sind Gegenstand der auf den Anspruch 1 zurückbezogenen Unteransprüche.

Das in der erfindungsgemäßen Vorrichtung betriebene Regelungsverfahren basiert im Wesentlichen darauf, dass zur Messung und Regelung der Gastemperatur am patientenseitigen Ende eines Heizschlauches ein mathematisches Modell herangezogen wird. Hierzu wird das Gesamtsystem bestehend aus Heizdraht, Messelektronik, Zuführleitung, Temperatursensor und Gasstrom durch einen Satz von Differenzialgleichungen beschrieben und in einem sogenannten Zustandsraummodell zusammengefasst. Unter der Voraussetzung, dass die Parameter des Modells hinreichend genau bestimmt sind, ergibt sich bei gleichen Eingangsgrößen eine Schätzung für die Gastemperatur am Gasschlauchausgang (d. h. Trokar-eingang). Durch einen Vergleich zwischen der tatsächlichen und der geschätzten Drahttemperatur können Abweichungen (sogenannte Beobachterfehler) detektiert werden. Diese können z. B. durch unterschiedliche Anfangszustände (z. B. liegen zu Beginn der Gaszufuhr keine a *priori* Informationen über die Gastemperatur vor)vorkommen. Wird der Beobachterfehler mit einem Gütekriterium bewertet und das Ergebnis anschließend auf das Modell zurückgeführt (Zustandsgrößenkorrektur), so klingt der Fehler ab und es ergibt sich im Ergebnis eine präzise Schätzung für die Gastemperatur am Schlauchausgang. Der Vorteil des vorgeschlagenen Verfahrens besteht unter anderem darin, dass für die Messung der Gastemperatur am Schlauchausgang kein zusätzlicher Temperatursensor benötigt wird. Im Ergebnis ergibt sich auch ohne Temperatursensor am Schlauchausgang eine Schätzgenauigkeit, die vergleichbar ist mit der Messgenauigkeit mittels eines herkömmlichen Schlauches, welcher einen Temperatursensor aufweist. Die Patientensicherheit ist somit auch ohne zusätzlichen Sensor gewährleistet.

Das erfindungsgemäße System ist in bevorzugter Weise so ausgestaltet, dass der Zustandsbeobachter, als Luenberger-Beobachter realisiert ist. Derartige Zustandsbeobachter inklusive der Luenberger-Beobachter sind z. B. in Lehrbüchern der Regelungstechnik erläutert.

Eine besondere Ausgestaltung einer derartigen Vorrichtung, die das oben beschriebene Verfahren realisiert, ist eine Insufflationseinrichtung für die Laparoskopie. Diese enthält eine Gaszufuhr (z. B. aus einer Druckflasche), die auf den nötigen Ausgangsdruck gebracht wird und zur Erzielung eines zweckmäßigen Volumenstroms eingerichtet ist. Der Volumenstrom ist dabei regelbar, z. B. zwischen 0 und 50 l/min. Über einen Zuführschlauch wird das Gas in das Körperinnere eingeführt. Zur Erzielung der gewünschten Temperatur (ca. Körpertemperatur, d. h. ca. 37°C) am Ausgang des Schlauches befindet sich im Inneren des Schlauches eine Heizeinrichtung, z. B. ein Heizdraht. Das in das Körperinnere eingeführte Gas, kann entweder durch separate Gasausgangsvorrichtungen, durch eine Absaugvorrichtung oder auch einfach durch Undichtigkeiten aus dem Körperinneren entweichen. Durch das oben beschriebene, erfindungsgemäße Verfahren wird die Ist-Temperatur am Schlauchausgang unter Verwendung der Messdaten aus dem Heizdraht (mittels Widerstandsmessung) geschätzt und durch Änderung der Heizleistung des Heizdrahtes geregelt. Dabei ist die Verwendung eines separaten Temperatursensors nicht nötig: Bei Verwendung eines Heizdrahtes, dessen Widerstand temperaturabhängig ist, kann die Messung der Heizdrahttemperatur über eine Widerstandsmessung erfolgen, so dass keine weiteren Bauteile erforderlich sind.

Eine alternative Ausführungsform der Erfindung besteht in einer Beatmungsvorrichtung. Durch die Beatmungsvorrichtung wird Sauerstoff bzw. sauerstoffhaltige Gasmischung in die Lunge des Patienten geleitet. Bei der Beatmung ist eine Befeuchtung des sauerstoffhaltigen Gasgemisches unabdingbar. Zur Vermeidung von Kondensation, wie auch zur Erzielung einer Gastemperatur, die für die Patienten angenehm ist, wird innerhalb des Beatmungsschlauches eine Widerstandsheizung über einen elektrischen Heizdraht realisiert. Analog zur oben beschriebenen Einrichtung für die Laparoskopie kann der Heizdraht über eine entsprechende Widerstandsmessung als Temperatursensor dienen. Die Ist-Temperatur am Schlauchausgang wird durch das erfindungsgemäße Verfahren geschätzt. Mittels des Schätzwertes wird die Heizleistung elektronisch geregelt. Im Ergebnis wird eine Vorrichtung erhalten, die eine präzise Messung und Regelung der Gastemperatur am Schlaucheingang gewährleistet, auch bei unterschiedlichsten Beatmungsbedingungen.

Ausführungsbeispiele der Erfindung sind in den Figuren dargestellt und werden nachfolgend näher erläutert:

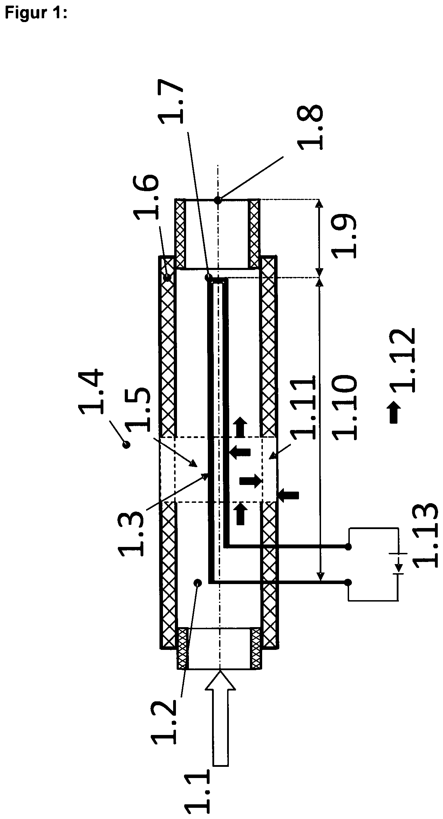

**Figur 1 zeigt modellhaft einen Gasversorgungsschlauch mit einem eingebauten Heizdraht, wobei die Bezugszeichen folgende Bedeutung haben:**

| | |
|---|---|
| 1.1 | Volumenstrom |
| 1.2 | *ϑ_{E}* Gaseintrittstemperatur |
| 1.3 | betrachtetes Heizdraht-Kontrollvolumen |
| 1.4 | *ξ* Umgebungstemperatur |
| 1.5 | betrachtetes Fluid-Kontrollvolumen |
| 1.6 | *η* Schlauchtemperatur |
| 1.7 | *σ*(*R_{Dr}*) Drahttemperatur |
| 1.8 | *ϑ* Gasaustrittstemperatur |
| 1.9 | unbeheizte Länge |
| 1.10 | beheizte Länge |
| 1.11 | betrachtetes Schlauch -Kontrollvolumen |
| 1.12 | ausgetauschte Wärmemengen |
| 1.13 | *U_{Dr}* Heizspannung |

Der Schlauch wird in Pfeilrichtung von einem Gasvolumenstrom durchströmt. Entsprechend der Länge des Heizdrahtes wird in diesem Modell aber nur eine Teillänge des Schlauches beheizt. Es folgen eine unbeheizte Restlänge und ein Lueradapter zum Übergang an den Patienten. Gemessen wird hierbei die Temperatur des Heizdrahtes per Widerstandsmessung. Geregelt werden soll die Temperatur der Strömung am Austritt aus dem Schlauch bei Volumenströmen von 0 - 50 l/min in einem Bereich von 32°C - 42°C.

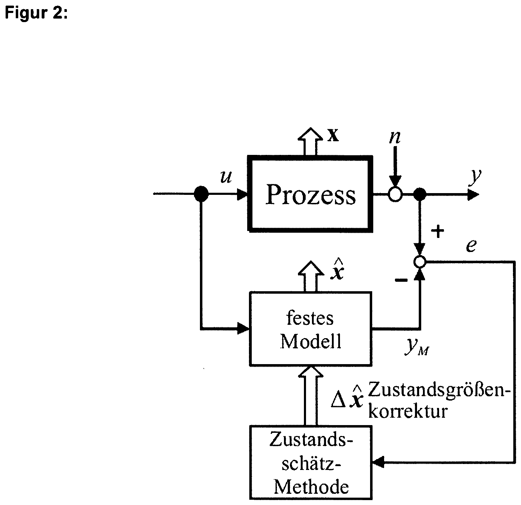

Im Rahmen des Verfahrens werden der Volumenstrom, die Temperatur des Heizdrahtes, die elektrische Leistung und deren zeitliche Verläufe kontinuierlich gemessen und verarbeitet. Figur 2 (aus Isermann R (2008). Mechatronische Systeme. Grundlagen. Springer-Verlag: Berlin) zeigt schematisch das erfindungsgemäße Schätzverfahren. Die Ansteuerung des Heizdrahtes erfolgt beispielsweise durch eine pulsweitenmodulierte Spannung (PWM). Gemessen wird die elektrische Leistung (U in Figur 2) und der Drahtwiderstand (Y in Figur 2). Die Messdaten werden in ein mathematisches Modell ("festes Modell" in Figur 2) übernommen, welches das dynamische Verhalten des Systems abbildet. Für diverse Durchflüsse sind unterschiedliche Modellparameter hinterlegt, sodass das Modell an den gemessenen Volumenstrom adaptiert werden kann. Der mittels des Modells geschätzte Temperaturwert wird mit dem gemessenen Ist-Wert der Drahttemperatur verglichen (y - y_{M} in Figur 2). Abweichungen zwischen dem Schätzwert und dem gemessenen Wert (e in Figur 2) werden so auf das Modell zurückgeführt, dass die Schätzung der Zustandsgrößen verbessert wird (Zustandsschätzmethode in Figur 2). Sobald die Schätzung mit dem Ist-Wert übereinstimmt, können die geschätzten Zustandsgrößen (*x̂* in Figur 2) übernommen und weiterhin angewandt werden. Eine dieser Zustandsgrößen ist die Austrittstemperatur der Gasströmung, welche im Ergebnis sehr exakt geschätzt wird.

Das erfindungsgemäße Verfahren weist eine Reihe von Vorteilen auf. Die beobachtete Temperatur/ Zustandsgröße berücksichtigt Störungen des Prozesses (Störgrößenbeobachter). Die beobachtete Größe kann als Regelgröße verwendet werden, so dass die Einstellung unterschiedlicher Sollwerte möglich ist. Insgesamt ergibt sich eine Regelungsleistung, die mit der möglichen Regelungsleistung bei Verwendung eines Temperatursensors (zur Messung der Strömungstemperatur) vergleichbar ist. Die Patientengefährdung wird dadurch weitestgehend ausgeschlossen und die Regelung lässt sich, durch den Wegfall des Strömungstemperatursensors, wesentlich kostengünstiger realisieren. Ein besonderer Vorteil des erfindungsgemäßen Verfahrens besteht darin, dass Fehler aufgrund defekter Strömungstemperatursensoren ausgeschlossen sind. Da in diesem Verfahren der Sensor und der Aktor identisch sind, fallen im Falle eines Defektes sowohl das Messglied als auch das Stellglied aus. Das Einbringen von Heizleistung ohne eine gleichzeitige Überprüfung durch Temperaturmessung ist nicht möglich.

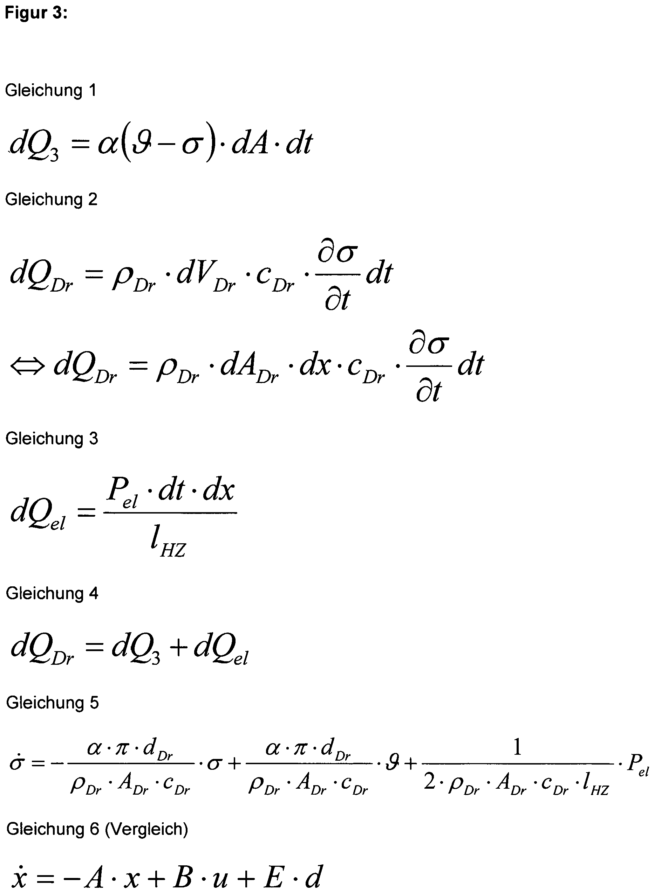

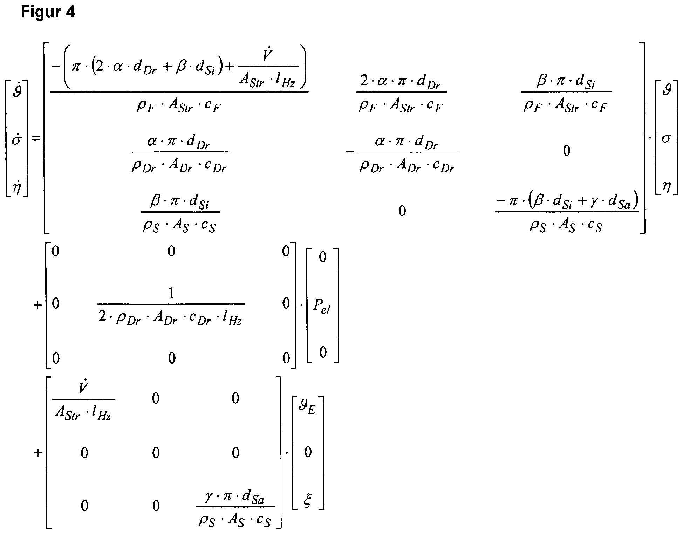

Zur Schätzung der Zustandsgröße (Austrittstemperatur) wird ein mathematisches Modell des Prozesses benötigt. Dieses mathematische Modell hat eine standardisierte Form, genannt Zustandsraummodell, welches in der Figur 4 wiedergebeben ist. Zur Ermittlung dieses Zustandsraummodells ist es notwendig ein physikalisches Ersatzmodell des Prozesses aufzustellen und in diese standardisierte Form zu bringen. Die aufgestellten Matrizen müssen mit Werten belegt werden (Identifikation). Die Vorgehensweise zur Beschreibung des zeitlichen Verhaltens der Drahttemperatur wird exemplarisch in Figur 3 gezeigt, wobei die zwischen Fluid und Draht ausgetauschte Wärmemenge (Gleichung 1), die im Draht gespeicherte Wärmemenge (Gleichung 2) und die zugeführte Wärmemenge in Form von Differenzialgleichungen (Gleichung 3) beschrieben ist. Die Gleichung 4 zeigt dann das Energiegleichgewicht (Wärmebilanz). Durch die Zusammenfassung der Gleichungen und geeignete Umformungen entsteht die Gleichung 5. Gleichung 6 zeigt zum Vergleich das angewandte Zustandsraummodell, welches mit Gleichung 6 weitgehend identisch ist und deren Koeffizienten die Parameter der Modellgleichungen enthalten. Entsprechend wird für die Modellierung der Gas- und Schlauchtemperatur (vgl. Figur 1) vorgegangen.

Figur 4 zeigt das sich ergebende Zustandsraummodell, welches vom Gasdurchfluss abhängig ist.

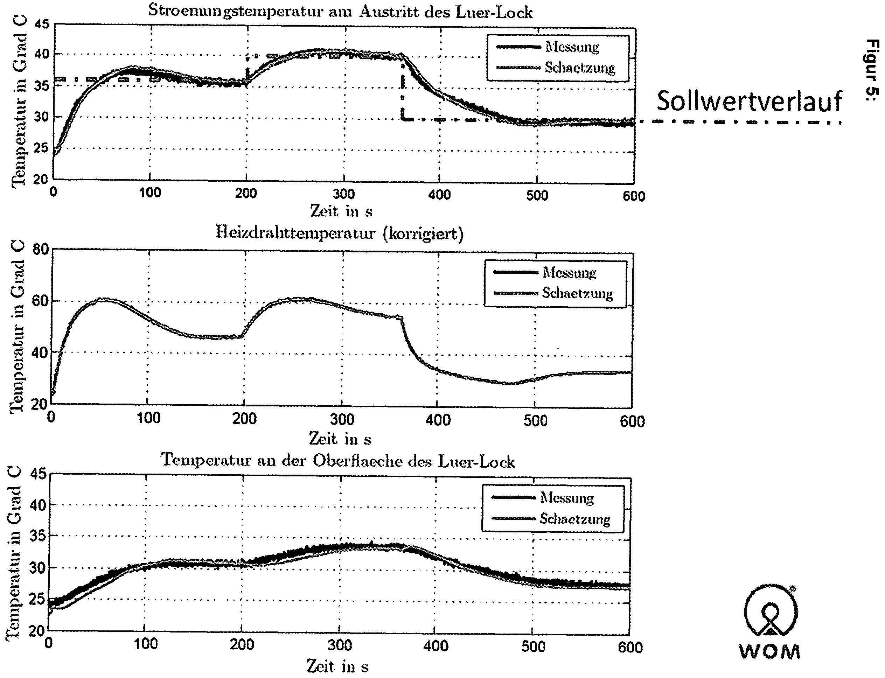

Figur 5 zeigt den Vergleich der tatsächlich gemessenen Daten mit den mittels des Verfahrens gewonnenen Schätzdaten. Im Ergebnis zeigt sich, dass das angewandte Modell korrekt ist und zu der notwendigen Genauigkeit der Schätzdatenführt.

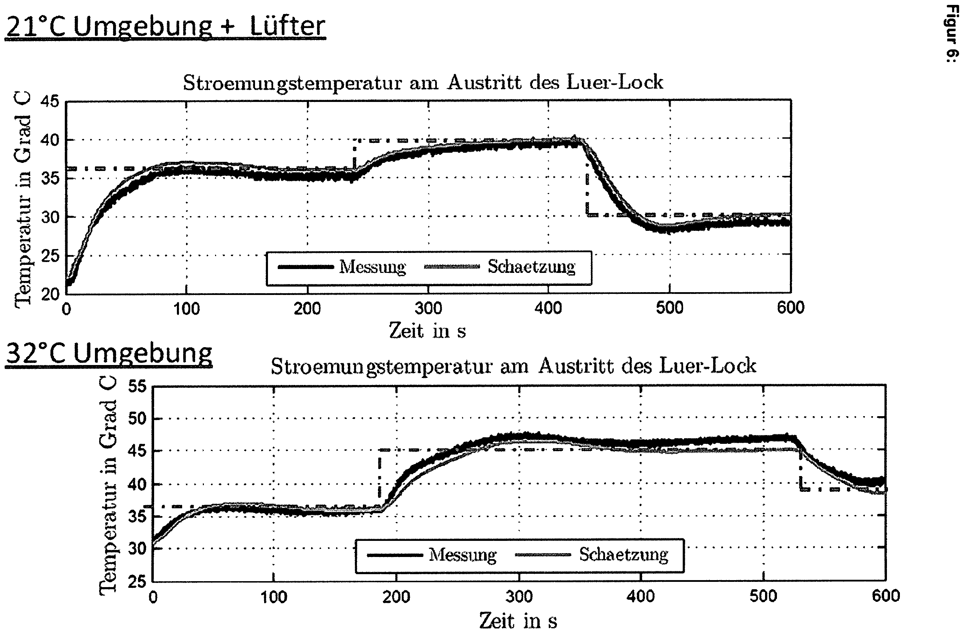

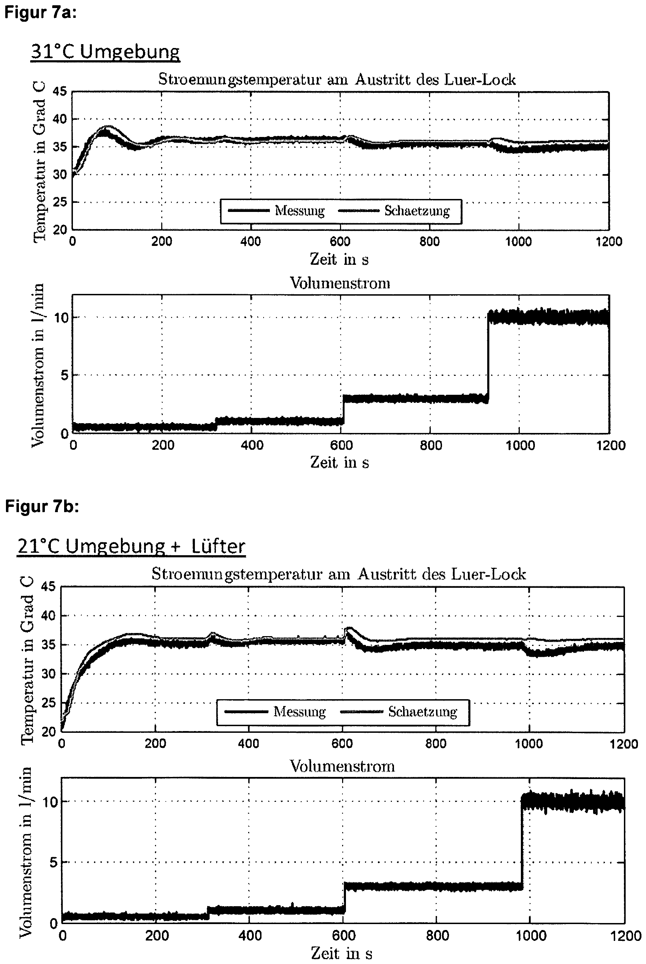

Figur 6 und 7 zeigen das Verfahren bei unterschiedlichen Umgebungsbedingungen, die als Störung modelliert wurden. Die reale Anwendung ist einer Reihe von Störungen unterworfen, wie z. B. unterschiedlicher Umgebungstemperatur (ξ in Figur 4) oder unterschiedlicher Gaseintrittstemperatur (ϑ_{E} in Figur 4). Die Störgrößen sind im Zustandsraummodell vorgesehen. Es zeigt sich eine hohe Übereinstimmung der gemessenen Temperatur mit der geschätzten Temperatur, auch bei Variation des Durchflusses.

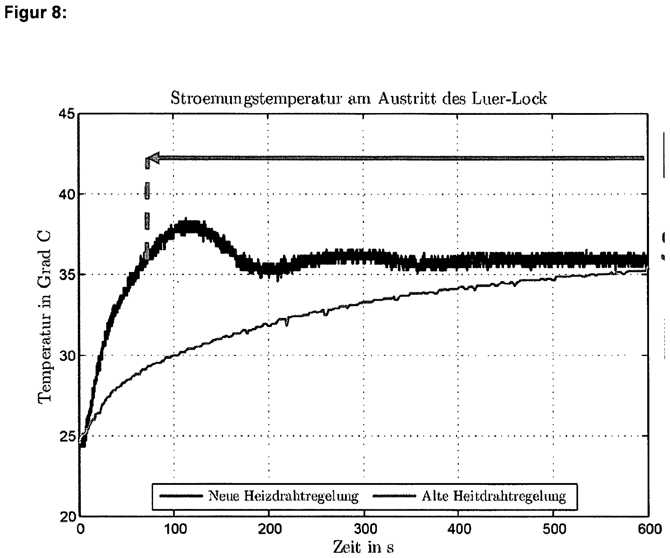

Figur 8 zeigt im Vergleich die erfindungsgemäße Heizdrahtregelung mit einer klassischen Vorsteuerung, die lediglich die Leistung des Heizdrahtes über den Widerstand des Heizdrahtes einstellt. Im Ergebnis ist zu sehen, dass das erfindungsgemäße Verfahren die Regelung sehr viel schneller bewerkstelligen kann.

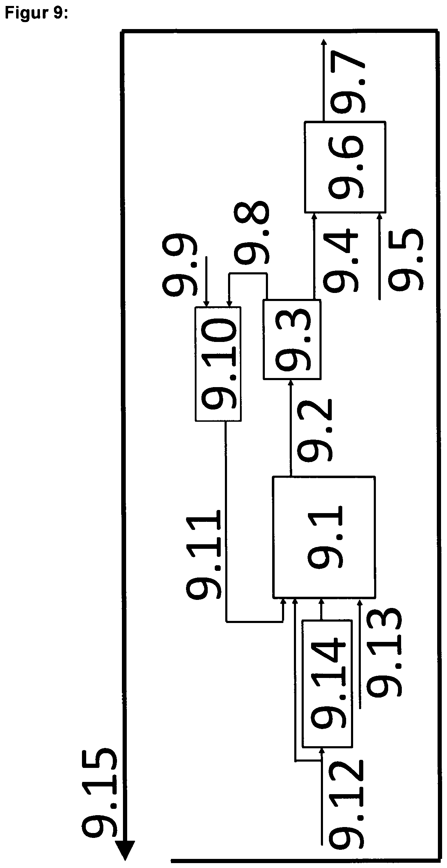

Die praktische Durchführung des oben beschriebenen Verfahrens erfolgt zweckmäßigerweise auf einem Mikrokontroller, der Bestandteil der medizintechnischen Vorrichtung ist. Diese ist üblicherweise mit Ein- und Ausgängen sowie Speichern versehen. Die mathematischen Operationen werden in Form eines Softwaremoduls abgearbeitet. Ein Ablaufdiagramm des Softwaremoduls ist in Figur 9 dargestellt, wobei die Bezugszeichen folgende Bedeutung haben:

| | |
|---|---|
| 9.1 | Numerische Lösung der Beobachterdifferentialgleichung |
| 9.2 | Geschätzte Zustandsgrößen |
| 9.3 | Separation der Zustandsgrößen |
| 9.4 | Geschätzte Gasaustrittstemperatur |
| 9.5 | Sollwert für Gasaustrittstemperatur |
| 9.6 | Regler |
| 9.7 | Heizdrahtspannung |
| 9.8 | Geschätzte Drahttemperatur |
| 9.9 | Gemessene Drahttemperatur |
| 9.10 | Berechnung Beobachterfehler |
| 9.11 | Beobachterfehler |
| 9.12 | Gemessener Volumenstrom |
| 9.13 | Gemessene Elektrische Leistung |
| 9.14 | Berechnung Korrekturvektor |
| 9.15 | Numerische Berechnung: i = i + 1 |

Die Software kann auf einem eigenen Speicherchip, z. B. eine EPROM hinterlegt sein.

Der Fachmann in dem Gebiet kann, basierend auf der vorliegenden Beschreibung, inklusive der Figuren und der zum Zeitpunkt der Anmeldung hinlänglich bekannten Fachliteratur innerhalb des Schutzbereiches der beigelegten Ansprüche weitere Ausführungsformen der Erfindung realisieren, ohne weiter erfinderisch tätig werden zu müssen.

## Patentansprüche

1. Medizintechnische Vorrichtung zum Einbringen von Gasen in Patienten enthaltend eine Gaszufuhrvorrichtung, einen Gaszufuhrschlauch, einen Heizdraht im Zuführschlauch, mindestens einen Mikroprozessor, mindestens einen Speicher und mindestens eine Software, wobei ein Gas durch die Gäszufuhrvorrichtung mittels des Gaszufuhrschlauch einem Patienten zugeführt wird,
wobei das Gas innerhalb des Gaszufuhrschlauches mittels des Heizdrahtes beheizt wird,
wobei die Heizleistung des Heizdrahtes elektrisch geregelt wird,
**dadurch gekennzeichnet, dass**
der Widerstand des Heizdrahtes eine Eingangsvariable eines mathematischen Schätzsystems nach Art eines Zustandsbeobachters ist, welcher mathematisch einen Zustandsraum beschreibt, welcher die tatsächliche Temperatur am Ausgang des Schlauches abschätzt und mittels dieses Schätzwertes die Heizleistung des Heizdrahtes regelt.

2. Vorrichtung gemäß Anspruch 1, **dadurch gekennzeichnet, dass** der Zustandsbeobachter nach Art eines Luenberger-Beobachters ausgebildet ist.

3. Vorrichtung gemäß Anspruch 1 oder 2, **dadurch gekennzeichnet, dass** das Gas CO₂ oder ein sauerstoffhaltiges Gasgemisch ist.

4. Medizintechnische Vorrichtung zum Einbringen von Gasen in Patienten gemäß Anspruch 1 bis 3, **dadurch gekennzeichnet, dass** es sich bei der Vorrichtung um einen Insufflator für die Laparoskopie handelt.

5. Medizintechnische Vorrichtung zum Einbringen von Gasen in Patienten gemäß Anspruch 1 bis 3, **dadurch gekennzeichnet, dass** es sich bei der Vorrichtung um eine Beatmungsvorrichtung handelt.

## Claims

1. A medical device for introducing gases into patients, comprising a gas supply device, a gas supply hose, a heating wire in the supply hose, at least one microprocessor, at least one memory and at least one software,
a gas being supplied by the gas supply device by means of the gas supply hose to a patient,
the gas being heated within the gas supply hose by means of the heating wire,
the heating power of the heating wire being electrically controlled,
**characterized by** that
the resistance of the heating wire is an input variable of a mathematical estimation system of the type of a state observer, which mathematically describes a state space, which estimates the actual temperature at the exit of the hose and controls the heating power of the heating wire by means of this estimated value.

2. The device according to claim 1, **characterized by** that the state observer is configured according to the type of a Luenberger observer.

3. The device according to claim 1 or 2, **characterized by** that the gas is CO₂ or an oxygen-containing gas mixture.

4. The medical device for introducing gases into patients according to claim 1 to 3, **characterized by** that the device is an insufflator for laparoscopy.

5. The medical device for introducing gases into patients according to claim 1 to 3, **characterized by** that the device is a breathing device.

## Revendications

1. Dispositif médical d'introduction de gaz dans des patients, comprenant un dispositif d'alimentation de gaz, un tuyau d'alimentation de gaz, une filière chauffante dans le tuyau d'alimentation, au moins un microprocesseur, au moins une mémoire et au moins un logiciel,
un gaz étant alimenté par le dispositif d'alimentation de gaz au moyen du tuyau d'alimentation de gaz à un patient,
le gaz étant chauffé au-dedans du tuyau d'alimentation de gaz au moyen de la filière chauffante,
la puissance de chauffage de la filière chauffante étant électriquement réglée,
**caractérisé en ce que**
la résistance de la filière chauffante est une variable d'entrée d'un système d'estimation mathématique du type d'observateur d'état, qui décrit par calcul un espace d'état, qui estime la température actuelle à la sortie du tuyau et règle la puissance de chauffage de la filière chauffante au moyen de cette valeur estimée.

2. Dispositif selon la revendication 1, **caractérisé en ce que** l'observateur d'état est configuré selon le type d'un observateur Luenberger.

3. Dispositif selon la revendication 1 ou 2, **caractérisé en ce que** le gaz est du CO₂ ou un mélange de gaz oxygénés.

4. Dispositif médical d'introduction de gaz dans des patients selon la revendication 1 à 3, **caractérisé en ce que** le dispositif est un insufflateur pour la laparoscopie.

5. Dispositif médical d'introduction de gaz dans des patients selon la revendication 1 à 3, **caractérisé en ce que** le dispositif est un appareil respiratoire.
